# EUROPÄISCHE PATENTSCHRIFT

(11) **EP 3 341 033 B1**
(45) Veröffentlichungstag und Bekanntmachung des Hinweises auf die Patenterteilung: **26.08.2026**
(21) Anmeldenummer: 16767141.1
(22) Anmeldetag: 01.08.2016
(51) Int. Cl.: A61L 2/18, A61B 90/70, A61L 2/24

(54) **VERFAHREN ZUM BETREIBEN EINER REINIGUNGS- UND DESINFEKTIONSEINRICHTUNG**
METHOD FOR OPERATING A CLEANING AND DISINFECTING DEVICE
PROCÉDÉ POUR FAIRE FONCTIONNER UN DISPOSITIF DE NETTOYAGE ET DE DÉSINFECTION

(30) Priorität: 27.08.2015 DE 102015216445
(43) Veröffentlichungstag der Anmeldung: 04.07.2018
(73) Patentinhaber: Olympus Winter & Ibe GmbH, 22045 Hamburg (DE)
(72) Erfinder: OTTENS, Benjamin, 22399 Hamburg (DE)
(74) Vertreter: Seemann & Partner Patentanwälte mbB
(86) Internationale Anmeldenummer: PCT/EP2016/068342
(87) Internationale Veröffentlichungsnummer: WO 2017/032561

(56) Entgegenhaltungen:
- EP-A1- 1 550 465
- EP-A1- 1 600 173
- EP-A2- 1 121 942
- WO-A1-2014/205366
- WO-A1-2015/040366
- WO-A1-2016/006270
- WO-A1-2016/087244
- WO-A2-2012/148868
- DE-A1- 102010 002 031
- JP-A- 2010 110 417
- US-A1- 2009 220 377
- US-A1- 2012 249 060
- US-A1- 2016 081 540

## Beschreibung

Die Erfindung betrifft ein Verfahren zum Betreiben einer Reinigungs- und Desinfektionseinrichtung, welche dazu eingerichtet ist, in einem validierten Reinigungs- und Desinfektionsvorgang ein chirurgisches Instrument, welches einer vorbestimmten Gruppe validierter chirurgischer Instrumente angehört, unter Verwendung zumindest eines Reinigungsmittels, welches einer vorbestimmten Gruppe validierter Reinigungsmittel angehört, in einem validierten Reinigungs- und Desinfektionsprozess aufzubereiten.

Reinigungs- und Desinfektionseinrichtungen für chirurgische Instrumente dienen dazu, sowohl die äußere Oberfläche der chirurgischen Instrumente als auch dessen Kanäle bzw. Kanalsystem zu reinigen und zu desinfizieren. Reinigungs- und Desinfektionseinrichtungen werden vielfach auch als Aufbereitungsvorrichtungen bezeichnet. Eine Reinigungs- und Desinfektionseinrichtung für Endoskope ist beispielsweise unter der Bezeichnung ETD (Endo-ThermoDesinfektor) des Herstellers Olympus Winter & Ibe GmbH, Hamburg, bekannt.

Aus WO 2014/205366 A1 ist eine Vorrichtung zum Desinfizieren und/oder Sterilisieren von medizinischer Kleidung oder Laborkleidung bekannt. Ein Desinfektionsschrank umfasst zur Nachverfolgung der Kleidung einen RFID-Leser. Die Kleidungsstücke sind mit RFID-Tags versehen, so dass diese identifiziert und auf ihrem Weg durch die Aufbereitung nachverfolgt werden können. Alternativ zu der Identifikation mittels RFID-Tags sind ein Barcode auf der Kleidung und ein zugehöriger Barcode-Leser auf Seiten der Desinfektionsvorrichtung vorgesehen.

WO 2015/040366 A1 offenbart ein Dekontaminierungssystem zum Reinigen und Desinfizieren eines medizinischen Instruments, wie beispielsweise eines Endoskops. Das System umfasst eine Vielzahl von Vorreinigungstüchern zum Abwischen des zu dekontaminierenden Instruments. Es handelt sich um ein System, bei dem nacheinander mehrere verschiedene Wischtücher zum Einsatz kommen. Jedes dieser Tücher ist einzeln verpackt und jede Verpackung ist mit einem maschinenlesbaren Code versehen. Um eine effiziente Reinigung sicherzustellen, werden nacheinander die Barcodes der einzelnen Desinfektionsstufen seitens des Systems angefordert.

Aus der nachveröffentlichten WO 2016/087244 A1 ist ein Aufbereitungssystem bekannt, bei dem in einer Gerätefront des Aufbereitungsgerätes ein Sensor vorhanden ist, der ein maschinenlesbares Kennzeichnungsmerkmal, beispielsweise einen RFID-Tag, an einem Reinigungskorb oder einem medizinischen Gerät erfasst. Sofern sich der RFID-Tag unmittelbar vor der Tür des Aufbereitungsgerätes befindet, wird automatisch ein Türöffnungssignal generiert, so dass der Zugang zu dem Aufbereitungsraum durch Öffnen der Tür freigegeben wird.

US 2012/0249060 A1 zeigt ein Autoklav zum Sterilisieren einer Batterie. Es ist vorgesehen, dass während einer ersten Betriebsphase die Temperatur der Batterie reduziert wird. Die Temperatur der Batterie steigt auf eine zweite Temperatur während einer zweiten Betriebsphase.

Eine Wasch- und Desinfektionsvorrichtung für Endoskope ist aus US 2009/0220377 A1 bekannt. Diese umfasst eine Fluidversorgungseinheit, die den Fluss der Spüllösung durch die einzelnen Endoskopkanäle mithilfe eines Flussmessers überwacht. Es ist vorgesehen, dass das Endoskop 2 mit einem RFID-Tag versehen ist, der von einem zugehörigen RFID-Leser der Wasch- und Desinfektionsvorrichtung ausgelesen wird. Es sind Spezifikationen für den auf diese Weise identifizierten Endoskoptyp hinterlegt, so dass die Endoskopkanäle mit der entsprechenden Flussrate gespült werden. Der Fluss durch die Endoskopkanäle wird von einem Flussmesser überwacht. Sollte der tatsächliche Fluss außerhalb vorgegebener Sollwerte liegen, wird eine entsprechende Fehlermeldung angezeigt.

JP 2010/110417 A1 zeigt ein System, bei dem eine Flasche mit Desinfektionslösung mit einem RFID-Tag versehen ist, der maschinenseitig von einem RFIF-Leser ausgelesen wird. So wird sichergestellt, dass eine vorgesehene Desinfektionslösung als Verbrauchsmaterial verwendet wird.

US 2016/0081540 offenbart eine Vorrichtung zum Reinigen und Desinfizieren von Endoskopen. Die Endoskope sind mit einem RFID-Tag versehen. Auf diesem ist beispielsweise der Typ oder eine Produktionsnummer gespeichert. Die Vorrichtung detektiert, ob das Endoskop in den Tank eingelegt oder aus diesem entfernt wird.

EP 1 600 173 A1 zeigt eine Kassette zur Aufnahme eines zu sterilisierenden Gegenstands. Diese Kassette ist für eine bestimmte Anzahl von Sterilisationszyklen ausgelegt. Um die Kassette identifizieren zu können, ist diese mit einem Barcode versehen, der auch durch einen RFIF-Tag ersetzt werden kann.

In WO 2012/148868 A2 ist ein Verfahren zur Verringerung des Verbrauchs eines Desinfektionsmittels offenbart. Es wird ein Desinfektionsmittel eingebracht, anschließend wird die Umgebung entfeuchtet, so dass sich die Desinfektionsmittelkonzentration auf der Oberfläche erhöht. Die Quelle des Desinfektionsmittels, beispielsweise ein Kanister, kann zur Identifikation des Desinfektionsmittels mit einem RFID-Tag versehen sein.

Ferner ist aus EP 1 121 942 A ein System bekannt, in dem ein flüssiges Sterilisationsmittel verdampft wird. Das System ist nicht funktionsfähig, wenn nicht der Behälter, in dem sich das Sterilisationsmittel befindet, durch Erfassen des Barcodes als zugelassen identifiziert wird.

DE 10 2010 002 031 A1 zeigt ein Gerät zum Desinfizieren, Sterilisieren und/oder Pflegen von zahnärztlichen Instrumenten. Ein eingesetztes Reinigungs- und Pflegemittel wird in einem Vorratsbehälter bereitgestellt, der mit einer entsprechenden Einschnürung oder Kerbe versehen ist. Nur derart ausgestaltete Behälter können in der Vorrichtung aufgenommen werden.

EP 1 550 465 A1 zeigt eine weitere Sterilisiervorrichtung für ein Endoskop. Sie umfasst eine RFID-Einheit, die mit einem RFID-Tag auf Seiten des Endoskops kommuniziert. Der Sterilisationsprozess wird entsprechend der von dem RFID-Tag des Endoskops ausgelesenen Informationen eingestellt.

Aus WO 2016/006270 A1 ist eine Kontrolleinheit bekannt, die verhindert, dass eine Endoskopaufbereitungsmaschine während des Aufbereitungsvorgangs versehentlich geöffnet wird.

An die Aufbereitung von chirurgischen Instrumenten, insbesondere Endoskopen, werden hohe hygienische Anforderungen gestellt. Um die hohen Anforderungen an die Qualität der Reinigung und Aufbereitung der chirurgischen Instrumente zu erfüllen und diese auch auf Dauer sicherzustellen, werden sowohl die Reinigungs- und Desinfektionseinrichtungen als auch der Reinigungsprozess selbst validiert. Ein solche Validierung findet typischerweise einmal jährlich statt.

Die Validierung des Aufbereitungsprozesses ist in Deutschland beispielsweise in der "Leitlinie zur Validierung maschineller Reinigungs- und Desinfektionsprozesse zur Aufbereitung thermolabiler Endoskope" im Detail festgeschrieben.

Im Rahmen der Validierung wird die Funktionalität der Reinigungs- und Desinfektionseinrichtungen, d.h. die Funktionalität der Maschine, festgestellt. Außerdem wird die Wirksamkeit des Aufbereitungsprozesses überprüft und validiert. Dies betrifft beispielsweise den Prozessablauf im Hinblick auf Spülblöcke, Temperaturen und Konzentration der verwendeten Reinigungschemikalien. Auch die verwendete Prozesschemie selbst wird im Rahmen der Validierung überprüft.

Schließlich umfasst die Validierung die Zuordnung zwischen dem Aufbereitungsprozess und dem zu reinigenden und zu desinfizierenden chirurgischen Instrument. Hierzu werden die in einem bestimmten Prozess aufzubereitenden chirurgischen Instrumente, beispielsweise Endoskope, einer bestimmten Gruppe zugeordnet. Für die Instrumente dieser Gruppe ist dann sichergestellt, dass im zugehörigen Aufbereitungsprozess die Reinigung und Desinfektion des jeweiligen Instruments mit der geforderten Qualität stattfindet.

Ausgehend von diesem Stand der Technik ist es eine Aufgabe der Erfindung, ein Verfahren zum Betreiben einer Reinigungs- und Desinfektionseinrichtung, eine Reinigungs- und Desinfektionseinrichtung sowie ein medizinisches System anzugeben, wobei der Reinigungs- und Desinfektionsprozess sicherer werden soll.

Die Aufgabe wird gelöst durch ein Verfahren zum Betreiben einer Reinigungs- und Desinfektionseinrichtung, nach Anspruch 1.

Das Verfahren gemäß Aspekten der Erfindung beruht auf den folgenden Überlegungen: Reinigungs- und Desinfektionseinrichtungen sind mit einer Vielzahl von Überwachungsfunktionen ausgestattet, die die korrekte Maschinenfunktionalität sicherstellen. Der Reinigungs- und Desinfektionsprozess (Aufbereitungsprozess) wird jedoch bisher nicht im Hinblick auf alle relevanten Parameter, welche im Rahmen einer Validierung verifiziert werden, überprüft. Hierbei handelt es sich um prozessrelevante Parameter, die von der Maschine selbst nicht beeinflusst werden. Bei diesen handelt es sich beispielsweise um die verwendeten Reinigungsmittel oder Reinigungschemikalien sowie um das aufzubereitende Endoskop, beispielsweise dessen Typ oder Baureihe. Um auf der Seite des Betreibers der Reinigungs- und Desinfektionseinrichtung sicherzustellen, dass die verwendeten Aufbereitungsprogramme, die verwendeten Reinigungsmittel und das aufzubereitende chirurgische Instrument, beispielsweise ein Endoskop, tatsächlich einem validierten Prozess entsprechen, wird die Zugehörigkeit des zu reinigenden chirurgischen Instruments und/oder die Zugehörigkeit des zu verwendenden Reinigungsmittels zu einer entsprechenden Gruppe validierter Einheiten überprüft. Im Rahmen dieser Überprüfung wird erkannt, ob seitens des Betreibers der Reinigungs- und Desinfektionseinrichtung alle notwendigen Maßnahmen definiert und umgesetzt sind, um die Aufbereitung in einem validierten Prozess sicherzustellen. Der Betreiber wird mit anderen Worten in die Lage versetzt, stets festzustellen, ob die Aufbereitung in einem validierten Prozess stattfindet oder nicht.

Das Verfahren zum Betreiben einer Reinigungs- und Desinfektionseinrichtung gemäß Aspekten der Erfindung ist somit sicherer als herkömmliche Verfahren.

Im Kontext der vorliegenden Beschreibung wird unter einer Validierung insbesondere eine Validierung des Aufbereitungsprozesses verstanden, wie sie in Deutschland entsprechend der "Leitlinie zur Validierung maschineller Reinigungs- und Desinfektionsprozesse zur Aufbereitung thermolabiler Endoskope" im Detail festgeschrieben ist. Diese Leitlinie ist beispielsweise in der Zeitschrift "Zentralsterilisation" in der Ausgabe 3/2011 als Supplement 3 veröffentlicht.

Das Verfahren zum Betreiben einer Reinigungs- und Desinfektionseinrichtung ist insbesondere dadurch fortgebildet, dass in dem Fall, in dem die Zugehörigkeit des zu reinigenden chirurgischen Instruments zu der validierten Gruppe chirurgischer Instrumente und/oder die Zugehörigkeit des zu verwendenden Reinigungsmittels zu der Gruppe validierter Reinigungsmittel und/oder die Validierung des Reinigungs- und Desinfektionsprozesses nicht positiv bestätigt werden, die Reinigungs- und Desinfektionseinrichtung eine Warnmitteilung generiert. Dabei ist insbesondere vorgesehen, dass überprüft wird, ob der Aufbereitungsprozess für die fragliche Gruppe chirurgischer Instrumente und/oder für die Gruppe von Reinigungsmitteln auch tatsächlich validiert ist. Beispielsweise wird sichergestellt, dass die Gültigkeitsdauer einer entsprechenden Validierung nicht überschritten ist.

Ferner ist insbesondere vorgesehen, dass die Warnmitteilung als Fehlermeldung von der Reinigungs- und Desinfektionseinrichtung ausgegeben und/oder in einem von der Reinigungs- und Desinfektionseinrichtung geführten Fehlerprotokoll als Fehlermeldung abgespeichert wird.

Beispielsweise gibt die Reinigungs- und Desinfektionseinrichtung eine entsprechende Warnung oder Fehlermeldung auf einem Display der Maschine aus. Der Benutzer ist in diesem Fall informiert, dass eine Aufbereitung in einem nicht validierten Prozess stattfindet. Ebenso ist vorgesehen, dass eine Warnung oder Fehlermeldung an einen zentralen Rechner kommuniziert wird. In der Reinigungs- und Desinfektionseinrichtung ist ferner beispielsweise vorgesehen, dass die Maschine ein öffentliches, d.h. für den Benutzer zugängliches, oder nicht öffentliches Fehlerprotokoll führt. In diesem werden die Fehlermeldungen optional abgespeichert.

Eine Gültigkeit der Validierung ist insbesondere eine zeitliche Gültigkeit. So wird der Aufbereitungsprozess beispielsweise einmal jährlich validiert und ist entsprechend für ein Jahr gültig. Anschließend muss eine neue Validierung durchgeführt werden. Es ist ebenso möglich, dass die Gültigkeit der Validierung von aktuellen Vorschriften abhängig ist. Werden beispielsweise die Vorschriften zur Aufbereitung chirurgischer Instrumente geändert, so verlieren Teile oder gegebenenfalls alle von der Reinigungs- und Desinfektionseinrichtung durchgeführten Aufbereitungsprozesse ihre Gültigkeit. Eine entsprechende Neuvalidierung ist notwendig.

Gemäß einer weiteren Ausführungsform ist das Verfahren dadurch fortgebildet, dass die Zugehörigkeit des zu reinigenden chirurgischen Instruments zu der validierten Gruppe chirurgischer Instrumente und/oder die Zugehörigkeit des zu verwendenden Reinigungsmittels zu der Gruppe validierter Reinigungsmittel und/oder die Gültigkeit der Validierung des Reinigungs- und Desinfektionsprozesses durch einen Abgleich mit in einer Datenbank der Reinigungs- und Desinfektionseinrichtung vorhandenen Einträgen erfolgt, wobei in dieser Datenbank Einträge betreffend alle chirurgischen Instrumente, die zu der validierten Gruppe chirurgischer Instrumente gehören, und/oder Einträge betreffend alle Reinigungsmittel, die zu der Gruppe validierter Reinigungsmittel gehören und/oder Einträge betreffend eine Gültigkeit der Validierung aller Reinigungs- und Desinfektionsprozesse der Reinigungs- und Desinfektionseinrichtung abgespeichert sind.

Die Datenbank ist insbesondere auf einem nichtflüchtigen Speicher der Reinigungs- und Desinfektionseinrichtung abgespeichert. Ein Abgleich wird beispielsweise von einer Steuereinheit der Reinigungs- und Desinfektionseinrichtung vorgenommen. Es ist insbesondere vorgesehen, dass die Datenbank per Fernzugriff eingespielt und/oder aktualisiert wird.

Eine nicht positive Bestätigung erfolgt, wenn beispielsweise ein Abgleich mit der Datenbank ergibt, dass das zu reinigende chirurgische Instrument in der Gruppe der validierten chirurgischen Instrumente nicht vorhanden ist. Ebenso erfolgt eine nicht positive Bestätigung, wenn der Validierungszeitraum für diese Gruppe von Instrumenten abgelaufen ist, beispielsweise weil die letzte Validierung länger als ein vorgegebenes Zeitintervall, beispielsweise ein Jahr, zurückliegt. Gleiches gilt auch für die Reinigungsmittel. Im Hinblick auf die Gültigkeit der Validierung des Reinigungs- und Desinfektionsprozesses erfolgt ebenfalls beispielsweise ein Abgleich mit einem in der Datenbank festgelegten Datum, zu dem die letzte Validierung des Prozesses durchgeführt wurde. Ein Vergleich dieses Zeitpunkts mit dem Zeitpunkt der durchzuführenden Aufbereitung, unter Berücksichtigung der vorgeschriebenen Gültigkeitsdauer, liefert das entsprechende Ergebnis.

Das Verfahren ist ferner insbesondere dadurch fortgebildet, dass die Reinigungs- und Desinfektionseinrichtung ein an dem chirurgischen Instrument und/oder an dem Reinigungsmittel vorhandenes maschinenlesbares Kennzeichnungsmerkmal ausliest, wobei das Kennzeichnungsmerkmal für das chirurgische Instrument und/oder für das Reinigungsmittel spezifische Informationen umfasst, die von der Reinigungs- und Desinfektionseinrichtung ausgelesen bzw. empfangen werden.

Als maschinenlesbares Kennzeichnungsmerkmal ist beispielsweise ein Barcode, ein QR-Code oder ein RFID-Tag vorgesehen. Hierzu umfasst das chirurgische Instrument beispielsweise einen entsprechenden RFID-Tag. Ebenso ist ein solches Kennzeichnungsmerkmal, beispielsweise ein RFID-Tag oder auch ein Barcode, an einem Kanister, in dem das Reinigungsmittel bereitgestellt wird, vorgesehen.

Die Reinigungs- und Desinfektionseinrichtung ist mit einem Bar-code- oder QR-Code-Leser oder einem RFID-Interrogator versehen, um die an dem chirurgischen Instrument oder an dem Reinigungsmittel vorhandenen Barcodes oder RFID-Tags auszulesen. Das maschinenlesbare Kennzeichnungsmerkmal umfasst beispielsweise Informationen hinsichtlich eines Typs und/oder einer Klassifikation des chirurgischen Instruments und/oder des Reinigungsmittels. Es ist ebenso vorgesehen, dass beispielsweise eine Serien- oder Chargennummer als Information hinterlegt ist. Die Bestimmung der Zugehörigkeit zu einer Gruppe, beispielsweise bei den chirurgischen Instrumenten, oder die Gültigkeit einer Validierung, beispielsweise bei den Reinigungsmitteln, wird seitens der Reinigungs- und Desinfektionseinrichtung anhand der Chargen- oder Produktionsnummer vorgenommen. Hierzu erfolgt beispielsweise ein Abgleich mit in der Datenbank vorhandenen Daten zu Chargen- oder Seriennummern.

Gemäß einer weiteren Ausführungsform ist das Verfahren dadurch fortgebildet, dass die Reinigungs- und Desinfektionseinrichtung das an dem chirurgischen Instrument und/oder an dem Reinigungsmittel vorhandene maschinenlesbare Kennzeichnungsmerkmal berührungslos ausliest.

Ein berührungslos arbeitendes System vereinfacht die Erkennung beispielsweise eines Kanisters mit Reinigungsmittel oder eines in die Reinigungs- und Desinfektionseinrichtung eingelegten chirurgischen Instruments. Der übliche Arbeitsablauf, beispielsweise das Einlegen der chirurgischen Instrumente in die Reinigungs- und Desinfektionseinrichtung oder das Auffüllen oder Ersetzen der Betriebsmittel/Reinigungsmittel, wird nicht verändert.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: ein vereinfachtes und schematisches Ablaufdiagramm eines Verfahrens zum Betreiben einer Reinigungs- und Desinfektionseinrichtung und
- Fig. 2: eine Reinigungs- und Desinfektionseinrichtung in einem medizinischen System in vereinfachter schematischer und perspektiver Darstellung.

In den Zeichnungen sind jeweils gleiche oder gleichartige Elemente und/oder Teile mit denselben Bezugsziffern versehen, so dass von einer erneuten Vorstellung jeweils abgesehen wird.

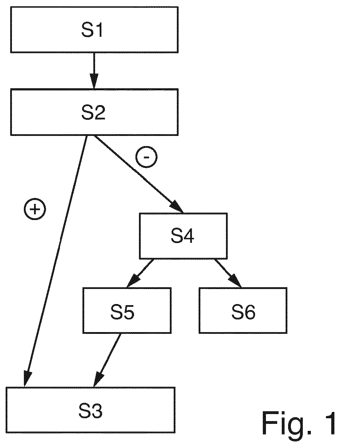

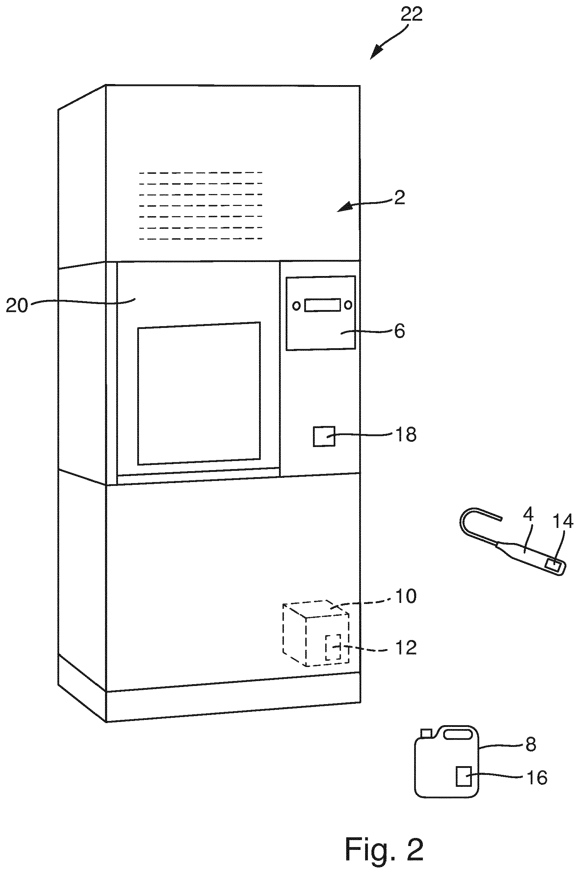

Fig. 1 zeigt ein vereinfachtes und schematisches Ablaufdiagramm eines Verfahrens zum Betreiben einer Reinigungs- und Desinfektionseinrichtung 2, wie sie beispielhaft Fig. 2 in vereinfachter, schematischer und perspektivischer Darstellung zeigt.

Die Reinigungs- und Desinfektionseinrichtung 2 ist dazu eingerichtet, ein chirurgisches Instrument 4, insbesondere ein Endoskop, in einem validierten Reinigungs- und Desinfektionsprozess aufzubereiten. Um sicherzustellen, dass das chirurgische Instrument 4 auch tatsächlich in einem validierten Reinigungs- und Desinfektionsprozess aufbereitet wird, wird seitens der Reinigungs- und Desinfektionseinrichtung 2 zu Beginn des Reinigungs- und Desinfektionsvorgangs, also bevor die Reinigung und Desinfektion des zu reinigenden chirurgischen Instruments 4 beginnt, überprüft, ob das chirurgische Instrument 4 zu einer validierten Gruppe chirurgischer Instrumente 4 gehört und/oder das zu verwendende Reinigungsmittel 8 zu einer Gruppe validierter Reinigungsmittel 8 gehört und/oder eine Validierung des Reinigungs- und Desinfektionsprozesses zum Zeitpunkt der Aufbereitung noch gültig ist. Es ist vorgesehen, dass die oben genannten Kriterien einzeln, in Kombination oder insbesondere kumulativ zur Anwendung kommen.

Das Verfahren zum Betreiben der Reinigungs- und Desinfektionseinrichtung 2 wird nun beispielhaft anhand von Fig. 1 erläutert. Zu Beginn des Aufbereitungsprozesses (Schritt S1), bevor der eigentliche Reinigungs- und Desinfektionsprozess startet, wird die oben genannte Überprüfung (Schritt S2) durchgeführt. Beispielsweise wird geprüft, ob das zu reinigende chirurgische Instrument 4 zu einer validierten Gruppe chirurgischer Instrumente 4 gehört. Außerdem wird geprüft, ob das zu verwendende Reinigungsmittel 8 zu einer Gruppe validierter Reinigungsmittel 8 gehört. Ferner wird überprüft, ob die Validierung des durchzuführenden Reinigungs- und Desinfektionsprozesses noch gültig ist. Werden alle diese Kriterien positiv bestätigt, so folgt das Verfahren dem mit "+" markierten Ast des Ablaufdiagramms. Das Reinigungs- und Desinfektionsprogramm startet in Schritt S3.

Wird hingegen eines der Kriterien nicht positiv bestätigt, so folgt das Verfahren dem mit "-" bezeichneten Ast. Es wird eine Warnmitteilung generiert (Schritt S4). Diese Warnmitteilung wird beispielsweise auf einer Ausgabeeinheit 6, z.B. einer Anzeige/Display, der Reinigungs- und Desinfektionseinrichtung 2 ausgegeben. Alternativ oder zusätzlich wird die Warnmitteilung in einem Fehlerprotokoll der Reinigungs- und Desinfektionseinrichtung 2 als Fehlermeldung abgespeichert (Schritt S5). Der Betreiber der Reinigungs- und Desinfektionseinrichtung 2 wird so in die Lage versetzt stets festzustellen, ob die Aufbereitung in einem validierten Prozess stattfindet oder nicht.

Alternativ ist vorgesehen, dass der Aufbereitungsprozess gestoppt wird bzw. erst gar nicht beginnt (Schritt S6). Gemäß diesem Ausführungsbeispiel verweigert die Reinigungs- und Desinfektionseinrichtung 2 den Betrieb, sollte die Möglichkeit bestehen, dass die aufzubereitenden chirurgischen Instrumente 4 in einem nicht validierten Prozess gereinigt und desinfiziert werden könnten.

Die zuvor beschriebene Funktionalität wird auf Seiten der Reinigungs- und Desinfektionseinrichtung 2 durch eine Steuereinheit 10 bereitgestellt. Diese umfasst beispielsweise eine Datenbank 12, die auf einem nicht flüchtigen Speichermedium der Steuereinheit 10 abgespeichert ist. Die Datenbank 12 umfasst Einträge betreffend alle chirurgischen Instrumente 4, die der validierten Gruppe chirurgischer Instrumente angehören. Hierbei handelt es sich beispielsweise um chirurgische Instrumente 4, beispielsweise Endoskope, einer bestimmten Baureihe oder eines bestimmten Typs, für die der Aufbereitungsprozess validiert ist. Ferner umfasst die Datenbank 12 Einträge betreffend alle Reinigungsmittel 8, die zu der Gruppe validierter Reinigungsmittel 8 gehören. Die Gruppe validierter Reinigungsmittel 8 umfasst beispielsweise Reinigungsmittel eines bestimmten Herstellers, für die der Aufbereitungsprozess validiert wurde. Ebenso sind in der Datenbank 12 Einträge vorhanden, die eine Gültigkeit der Validierung aller Reinigungs- und Desinfektionsprozesse oder -programme der Reinigungs- und Desinfektionseinrichtung 2, insbesondere im Hinblick auf ihre zeitliche Gültigkeit, angeben. Durch einen Abgleich der Daten des zu reinigenden chirurgischen Instruments 4 bzw. des für die Aufbereitung zu verwendenden Reinigungsmittels 8 und des vorgesehenen Reinigungs- und Desinfektionsprogramms (unter Berücksichtigung des aktuellen Datums) mit den entsprechenden Einträgen der Datenbank 12 ist es möglich, eine Überprüfung der zuvor genannten Kriterien vorzunehmen. Sofern die Validierung jeweils gültig ist, wird der Reinigungs- und Desinfektionsprozess positiv bestätigt und die Aufbereitung beginnt.

Gemäß einem Ausführungsbeispiel sind das chirurgische Instrument 4 und/oder das Reinigungsmittel 8 jeweils mit einem maschinenlesbaren Kennzeichnungsmerkmal 14, 16 versehen. Das entsprechende maschinenlesbare Kennzeichnungsmerkmal 14, 16 umfasst Informationen, welche für das aufzubereitende chirurgische Instrument 4 bzw. für das zu verwendende Reinigungsmittel 8 spezifisch sind. Beispielsweise sind in dem maschinenlesbaren Kennzeichnungsmerkmal 14, 16 Informationen betreffend einen Typ, eine Seriennummer, einen Hersteller oder dgl. hinterlegt.

Bei dem maschinenlesbaren Kennzeichnungsmerkmal 14, 16 handelt es sich beispielsweise um einen Bar-Code, einen QR-Code und insbesondere um einen RFID-Tag. Um einen RFID-Tag auszulesen, umfasst die Reinigungs- und Desinfektionseinrichtung 2 einen Sensor 18. So wird vorteilhaft die Möglichkeit geschaffen, eine berührungslose Erkennung des chirurgischen Instruments 4 bzw. des zu verwendenden Reinigungsmittels 8 durchzuführen. Eine berührungslose Erkennung ist besonders vorteilhaft, da der übliche Arbeitsablauf durch diesen Schritt nicht unterbrochen wird. So kann beispielsweise - wie üblich - das aufzubereitende chirurgische Instrument 4 in einen Reinigungskorb (nicht dargestellt) eingelegt werden und dieser Reinigungskorb nach Öffnung der Tür 20 der Reinigungs- und Desinfektionseinrichtung 2 in deren Innenraum eingebracht werden. Sobald das vorgesehene Reinigungs- und Desinfektionsprogramm gestartet wird, findet zunächst eine Erkennung der im Innenraum der Reinigungs- und Desinfektionseinrichtung 2 vorhandenen chirurgischen Instrumente 4 statt. Eine Gültigkeit der entsprechenden Validierung des vorgesehenen Reinigungsprozesses findet statt. In ähnlicher Weise wird auch der Wechsel oder das Befüllen der Reinigungs- und Desinfektionseinrichtung 2 mit Reinigungsmitteln 8 gegenüber dem herkömmlichen Prozess praktisch nicht verändert. Wird beispielsweise ein Kanister mit neuem Reinigungsmittel 8 in die Reinigungs- und Desinfektionseinrichtung 2 eingebracht, so wird nach Erkennung des neuen Kanisters automatisch der auf dem Kanister des Reinigungsmittels 8 vorhandene RFID-Tag als maschinenlesbares Kennzeichnungsmerkmal 16 ausgelesen. Die Steuereinheit 10 der Reinigungs- und Desinfektionseinrichtung 2 wird so in die Lage versetzt, beispielsweise durch einen Abgleich mit auf der Datenbank 12 vorhandenen Datensätzen, festzustellen, ob das ersetzte Reinigungsmittel 8 zu einem validierten Aufbereitungsprozess gehört oder nicht.

Im Ergebnis wird in einem medizinischen System 22, wie es vereinfacht und schematisch Fig. 2 zeigt, und welches neben der Reinigungs- und Desinfektionseinrichtung 2 zumindest ein zu verwendendes Reinigungsmittel 8 und zumindest ein zu reinigendes medizinisches Instrument 4 umfasst, die Aufbereitung, d.h. die Reinigung und Desinfektion des chirurgischen Instruments 4, im Hinblick auf die Prozesssicherheit wesentlich verbessert.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: Reinigungs- und Desinfektionseinrichtung
- 4: chirurgisches Instrument
- 6: Ausgabeeinheit
- 8: Reinigungsmittel
- 10: Steuereinheit
- 12: Datenbank
- 14, 16: maschinenlesbares Kennzeichnungsmerkmal
- 18: Sensor
- 20: Tür
- 22: medizinisches System

## Patentansprüche

1. Verfahren zum Betreiben einer Reinigungs- und Desinfektionseinrichtung (2), welche dazu eingerichtet ist, ein chirurgisches Instrument (4), welches einer vorbestimmten Gruppe validierter chirurgischer Instrumente (4) angehört, unter Verwendung zumindest eines Reinigungsmittels (8), welches einer vorbestimmten Gruppe validierter Reinigungsmittel (8) angehört, in einem validierten Reinigungs- und Desinfektionsprozess aufzubereiten, **dadurch gekennzeichnet, dass** die Reinigungs- und Desinfektionseinrichtung (2) zu Beginn des Reinigungs- und Desinfektionsvorgangs, bevor die Aufbereitung eines aufzubereitenden chirurgischen Instruments (4) beginnt, eine Zugehörigkeit des zu reinigenden chirurgischen Instruments (4) zu der validierten Gruppe chirurgischer Instrumente (4) und die Zugehörigkeit eines zu verwendenden Reinigungsmittels (8) zu der Gruppe validierter Reinigungsmittel (8) und eine Gültigkeit einer Validierung des Reinigungs- und Desinfektionsprozesses überprüft.

2. Verfahren nach Anspruch 1, **dadurch gekennzeichnet, dass** in dem Fall, in dem die Zugehörigkeit des aufzubereitenden chirurgischen Instruments (4) zu der validierten Gruppe chirurgischer Instrumente (4) und die Zugehörigkeit des zu verwendenden Reinigungsmittels (8) zu der Gruppe validierter Reinigungsmittel (8) und die Validierung des Reinigungs- und Desinfektionsprozesses für die Gruppe chirurgischer Instrumente (14) und die Gruppe von validierten Reinigungsmitteln (8) nicht positiv bestätigt werden, die Reinigungs- und Desinfektionseinrichtung eine Warnmitteilung generiert.

3. Verfahren nach Anspruch 2, **dadurch gekennzeichnet, dass** die Warnmitteilung als Fehlermeldung von der Reinigungs- und Desinfektionseinrichtung (2) ausgegeben und/oder in einem von der Reinigungs- und Desinfektionseinrichtung (2) geführten Fehlerprotokoll als Fehlermeldung abgespeichert wird.

4. Verfahren nach einem der Ansprüche 1 bis 3, **dadurch gekennzeichnet, dass** die Zugehörigkeit des zu reinigenden chirurgischen Instruments (4) zu der validierten Gruppe chirurgischer Instrumente (4) und die Zugehörigkeit des zu verwendenden Reinigungsmittels (8) zu der Gruppe validierter Reinigungsmittel (8) und die Gültigkeit der Validierung des Reinigungs- und Desinfektionsprozesses durch einen Abgleich mit in einer Datenbank (12) der Reinigungs- und Desinfektionseinrichtung (2) vorhandenen Einträgen erfolgt, wobei in dieser Datenbank (12) Einträge betreffend alle chirurgischen Instrumente (4), die zu der validierten Gruppe chirurgischer Instrumente (4) gehören, und Einträge betreffend alle Reinigungsmittel (8), die zu der Gruppe validierter Reinigungsmittel (8) gehören und Einträge betreffend eine Gültigkeit der Validierung aller Reinigungs- und Desinfektionsprozesse der Reinigungs- und Desinfektionseinrichtung (2) abgespeichert sind.

5. Verfahren nach einem der Ansprüche 1 bis 4, **dadurch gekennzeichnet, dass** die Reinigungs- und Desinfektionseinrichtung (2) ein an dem chirurgischen Instrument (4) und/oder an dem Reinigungsmittel (8) vorhandenes maschinenlesbares Kennzeichnungsmerkmal (14, 16) ausliest, wobei das Kennzeichnungsmerkmal (14, 16) für das chirurgische Instrument (4) und/oder für das Reinigungsmittel (8) spezifische Informationen umfasst, die von der Reinigungs- und Desinfektionseinrichtung (2) ausgelesen bzw. empfangen werden.

6. Verfahren nach Anspruch 5, **dadurch gekennzeichnet, dass** die Reinigungs- und Desinfektionseinrichtung (2) das an dem chirurgischen Instrument (4) und/oder an dem Reinigungsmittel (8) vorhandene maschinenlesbare Kennzeichnungsmerkmal (14, 16) berührungslos ausliest.

## Claims

1. A method for operating a cleaning and disinfecting device (2) which is designed to treat a surgical instrument (4) which belongs to a predetermined group of validated surgical instruments (4), using at least one cleaning agent (8) which belongs to a predetermined group of validated cleaning agents (8), in a validated cleaning and disinfecting process, **characterized in that** the cleaning and disinfecting device (2) checks, at the start of the cleaning and disinfecting operation, before the treatment of a surgical instrument (4) to be treated begins, whether the surgical instrument (4) to be cleaned belongs to the validated group of surgical instruments (4) and whether a cleaning agent (8) to be used belongs to the group of validated cleaning agents (8) and whether a validation of the cleaning and disinfecting process is valid.

2. The method according to Claim 1, **characterized in that** in the case in which the question of whether the surgical instrument (4) to be treated belongs to the validated group of surgical instruments (4) and the question of whether the cleaning agent (8) to be used belongs to the group of validated cleaning agents (8), and the validation of the cleaning and disinfecting process for the group of surgical instruments (14) and the group of validated cleaning agents (8), are not positively confirmed, the cleaning and disinfecting device generates a warning message.

3. The method according to Claim 2, **characterized in that** the warning message is output as an error message by the cleaning and disinfecting device (2) and/or is stored as an error message in an error log kept by the cleaning and disinfecting device (2).

4. The method according to any one of Claims 1 to 3, **characterized in that** the question of whether the surgical instrument (4) to be cleaned belongs to the validated group of surgical instruments (4) and the question of whether the cleaning agent (8) to be used belongs to the group of validated cleaning agents (8) and the question of whether the validation of the cleaning and disinfecting process is valid are checked by a comparison with entries present in a database (12) of the cleaning and disinfecting device (2), wherein entries regarding all of the surgical instruments (4) which belong to the validated group of surgical instruments (4), and entries regarding all of the cleaning agents (8) which belong to the group of validated cleaning agents (8), and entries regarding a validity of the validation of all of the cleaning and disinfecting processes of the cleaning and disinfecting device (2), are stored in said database (12).

5. The method according to any one of Claims 1 to 4, **characterized in that** the cleaning and disinfecting device (2) reads out a machine-readable characterizing feature (14, 16) which is present on the surgical instrument (4) and/or on the cleaning agent (8), wherein the characterizing feature (14, 16) for the surgical instrument (4) and/or for the cleaning agent (8) comprises specific information which is read out or, respectively received by the cleaning and disinfecting device (2).

6. The method according to Claim 5, **characterized in that** the cleaning and disinfecting device (2) reads out the machine-readable characterizing feature (14, 16) which is present on the surgical instrument (4) and/or on the cleaning agent (8) contactlessly.

## Revendications

1. Procédé pour faire fonctionner un dispositif (2) de nettoyage et de désinfection conçu pour traiter un instrument chirurgical (4) appartenant à un groupe prédéterminé d'instruments chirurgicaux validés (4) en utilisant au moins un produit de nettoyage (8), appartenant à un groupe prédéterminé de produits de nettoyage validés (8), dans le cadre d'un processus de nettoyage et de désinfection validé, **caractérisé en ce que** le dispositif (2) de nettoyage et de désinfection, au début du processus de nettoyage et de désinfection, avant que le traitement d'un instrument chirurgical (4) à traiter, vérifie l'appartenance de l'instrument chirurgical (4) à nettoyer au groupe validé d'instruments chirurgicaux (4), l'appartenance d'un agent de nettoyage (8) à utiliser au groupe d'agents de nettoyage validés (8) et la validité d'une validation du processus de nettoyage et de désinfection.

2. Procédé selon la revendication 1, **caractérisé en ce que**, dans le cas où l'appartenance de l'instrument chirurgical (4) à retraiter au groupe validé d'instruments chirurgicaux (4) et l'appartenance du produit de nettoyage (8) à utiliser au groupe de produits de nettoyage validés (8) ainsi que la validation du processus de nettoyage et de désinfection pour le groupe d'instruments chirurgicaux (14) et le groupe de produits de nettoyage validés (8) ne sont pas confirmées de manière positive, le dispositif de nettoyage et de désinfection génère un message d'avertissement.

3. Procédé selon la revendication 2, **caractérisé en ce que** le message d'avertissement est émis sous la forme d'un message d'erreur par le dispositif (2) de nettoyage et de désinfection et/ou est enregistré en tant que message d'erreur dans un journal des erreurs tenu par le dispositif (2) de nettoyage et de désinfection.

4. Procédé selon l'une des revendications 1 à 3, **caractérisé en ce que** l'appartenance de l'instrument chirurgical (4) à nettoyer au groupe validé d'instruments chirurgicaux (4) et l'appartenance du produit de nettoyage (8) à utiliser au groupe de produits de nettoyage validés (8) ainsi que la validité de la validation du processus de nettoyage et de désinfection s'effectuent par une comparaison avec des entrées présentes dans une base de données (12) du dispositif (2) de nettoyage et de désinfection, cette base de données (12) contenant des entrées enregistrées concernant tous les instruments chirurgicaux (4) appartenant au groupe validé d'instruments chirurgicaux (4), ainsi que des entrées concernant tous les produits de nettoyage (8) appartenant au groupe de produits de nettoyage validés (8) et des entrées concernant la validité de la validation de tous les processus de nettoyage et de désinfection du dispositif (2) de nettoyage et de désinfection.

5. Procédé selon l'une des revendications 1 à 4, **caractérisé en ce que** le dispositif (2) de nettoyage et de désinfection lit une caractéristique d'identification (14, 16) lisible par machine présente sur l'instrument chirurgical (4) et/ou sur le produit de nettoyage (8), la caractéristique d'identification (14, 16) comprenant des informations spécifiques à l'instrument chirurgical (4) et/ou au produit de nettoyage (8), qui sont lues ou reçues par le dispositif (2) de nettoyage et de désinfection.

6. Procédé selon la revendication 5, **caractérisé en ce que** le dispositif (2) de nettoyage et de désinfection lit sans contact la caractéristique d'identification (14, 16) lisible par machine présente sur l'instrument chirurgical (4) et/ou sur le produit de nettoyage (8).
